# EUROPÄISCHE PATENTSCHRIFT

(11) **EP 1 139 980 B1**
(45) Veröffentlichungstag und Bekanntmachung des Hinweises auf die Patenterteilung: **21.02.2007**
(21) Anmeldenummer: 99965416.3
(22) Anmeldetag: 30.11.1999
(51) Int. Cl.: A61K 8/00, A61Q 1/00, A61Q 19/00

(54) **KOSMETISCHE ODER MEDIZINISCHE ZUBEREITUNG FÜR DIE TOPISCHE ANWENDUNG**
COSMETIC OR MEDICAL PREPARATION FOR TOPICAL USE
PREPARATIONS COSMETIQUES OU MEDICALES POUR UTILISATION TOPIQUE

(30) Priorität: 28.12.1998 DE 19860371
(43) Veröffentlichungstag der Anmeldung: 10.10.2001
(73) Patentinhaber: Südzucker Aktiengesellschaft Mannheim/Ochsenfurt, 68165 Mannheim (DE)
(72) Erfinder: BENGS, Holger, D-60598 Frankfurt (DE); SCHNELLER, Arnold, D-64409 Messel (DE); GRANDE, Jürgen, D-65812 Bad Soden (DE); SCHUTH, Silke, D-56412 Ruppach-Goldhausen (DE); BÖHM, Gitte, D-60439 Frankfurt (DE); BRAUNAGEL, Alfred, D-55128 Mainz (DE)
(74) Vertreter: Luderschmidt, Schüler & Partner
(86) Internationale Anmeldenummer: PCT/EP1999/009293
(87) Internationale Veröffentlichungsnummer: WO 2000/038623

(56) Entgegenhaltungen:
- WO-A-00/12589
- WO-A-00/12617
- WO-A-95/31553
- WO-A-97/28780
- WO-A-99/11695
- DE-A- 4 120 760
- DE-C- 19 839 216
- FR-A- 2 747 306
- GB-A- 2 247 242
- US-A- 5 726 161
- INABA, R. ET AL.: "J. SCCJ (1995), 29(2), p.146-153: Application of porous starch complex powder" STN INTERNATIONAL; FILE CAPLUS, AN1995:883105, XP002130221
- MIYOSHI, R.; KISHIDA,S.: "Body powder containing menthol" STN INTERNATIONAL; FILE CAPLUS, AN1986:613944, XP002135215

## Beschreibung

Die vorliegende Erfindung betrifft eine kosmetische oder medizinische Zubereitung für die topische Anwendung, insbesondere Hautpflegemittel, enthaltend sphärische Mikropartikel, die ganz oder teilweise aus mindestens einem linearen wasserunlöslichen Polyglucan bestehen, sowie die Verwendung von sphärischen Mikropartikeln, die ganz oder teilweise aus mindestens einem linearen wasserunlöslichen Polyglucan bestehen, in derartigen Zubereitungen. Insbesondere betrifft die vorliegende Erfindung eine kosmetische oder medizinische Zubereitung für die topische Anwendung, die beim Auftragen ein besonders angenehmes weiches Gefühl vermittelt.

Die Verwendung von Polysacchariden auf Stärkebasis wie Polyglucanen für kosmetische Zwecke ist seit altersher bekannt.

In der letzten Zeit wurden zunehmend Polysaccharidprodukte für kosmetische und therapeutische Zwecke entwickelt, die spezielle Eigenschaftsprofile aufweisen.
So wird von H. Eggensperger, M. Wilker in SÖFW-Joumal, 123. Jahrgang 8/97, Seiten 542 bis 546, "Multiaktivwirksame Polysaccharide, Teil I - Pilzextrakte" für beta-Polyglucane aus Pilzen wie Hefen und deren carboxymethylierte Derivate ein hohe pflegende Wirkung für gereizte, trockene Haut beschrieben.
Besonders vorteilhafte Effekte auf die Haut konnten für beta-1,3-Polyglucane mit beta-1,6- Verknüpfungen nachgewiesen werden, für die zudem eine immunstimulierende Wirkung und Tumoraktivität beobachtet wurde (o.a. Ort, F. Zülli et al., am o.a. Ort, Seiten 535 bis 541).

Auch Cyclodextrine, cyclische alpha-, beta- oder gamma 1,4-Oligosaccharide mit 6 bis 8 Glucaneinheiten, finden steigend Anwendung für die funktionelle Hautpflege, da sie mit einer Vielzahl von Wirkstoffen und pflegenden Substanzen Einschlußverbindungen bilden und dadurch eine retardierte Freisetzung dieser Substanzen am Anwendungsort bewirken können (U. Citemesi, M. Scaiacchitano in Cosmetics and Toiletries magazine, Band 110, März 1995, Seiten 53 bis 61 "Cyclodextrines in functional Dermocosmetics").

Die GB-A-2247242 offenbart sphärische Teilchen aus entzweigter Stärke.

Obwohl eine Vielzahl von Produkten für die unterschiedlichsten kosmetischen und medizinischen Verwendungszwecke bekannt sind, besteht ein ständiger Bedarf nach neuen verbesserten Produkten. Wünschenswert sind insbesondere Produkte, die beim Auftragen auf die Haut ein angenehmes Gefühl erzeugen und einen glatten, weichen Eindruck vermitteln.

Derartige Produkte sind vorteilhaft für die Anwendung bei empfindlicher Haut und eignen sich speziell auch für die Herstellung von medizinischen Zubereitungen für die topische Applikation.

Es hat sich gezeigt, daß Zubereitungen für die topische Anwendung (nachfolgend auch topische Zubereitung genannt) beim Auftragen ein besonders angenehmes, weiches Gefühl vermitteln, wenn ihnen sphärische Mikropartikel zugesetzt werden, die ganz oder teilweise aus linearen wasserunlöslichen Polyglucanen bestehen.

Erfindungsgemäß wird somit eine Zubereitung zur topischen Anwendung zur Verfügung gestellt, die sphärische Mikropartikel enthält, die ganz oder teilweise aus mindestens einem linearen wasserunlöslichen Polyglucan bestehen, wobei linear einen Verzweigungsgrad vom nicht mehr als 0,5% in 6-Position bedeutet.

Unter Zubereitungen für die topische Anwendung werden im Sinne der Erfindung ganz allgemein kosmetische Mittel also Körperpflegemittel und dekorative Kosmetika verstanden. Erfindungsgemäß umfaßt der Begriff auch human- und tiermedizinische Zubereitungen, die äußerlich appliziert werden.

Erfindungsgemäße Zubereitungen können Cremes, Kompaktcremes, Lotionen, Masken, Puder in beliebiger Form z.B. flüssig, lose, kompakt, partikelförmig etc., Salben, Salbengrundlagen, Seifen usw. für die dekorative, pflegende oder medizinische Anwendung sein.

Beispiele für die dekorative Kosmetik sind Cremes, Puder, oder Fonds für Make-up, z.B. Rouge, Lidschatten, Lippenstifte.

Wesentlich für die Lösung dieser Aufgabe ist der erfindungsgemäße Einsatz der sphärischen Mikropartikel, die ganz oder teilweise aus mindestens einem wasserunlöslichen linearen Polyglucan bestehen.

Derartige Mikropartikel zeichnen sich durch eine hohe Gleichförmigkeit in Größe und Gestalt aus. Zudem können sie selbst ohne Zusatz von Dispergierhitfsmittel leicht stabile Suspensionen ausbilden, was besonders vorteilhaft für die Herstellung der erfindungsgemäßen Zubereitungen ist, soweit sie auf Emulsionsbasis beruhen.

Weiter zeichnen sich die erfindungsgemäß eingesetzten Mikropartikel durch eine hohe Biokompatibilität aus.

Für die Biokompatibilität der erfindungsgemäß eingesetzten Mikropartikel ist insbesondere der naturidentische Charakter der für die Herstellung verwendeten wasserunlöslichen linearen Polyglucane sowie von deren Abbauprodukten von hoher Bedeutung.

Unter sphärischen Mikropartikeln sind Mikropartikel zu verstehen, die annähernd Kugelform besitzen. Bei Beschreibung einer Kugel durch von einem gemeinsamen Ursprung ausgehende, in den Raum gerichtete Achsen gleicher Länge, die den Radius der Kugel in allen Raumrichtungen definieren, ist für die sphärischen Partikel eine Abweichung der Achsenlängen vom Idealzustand der Kugel von 1 % bis 40 % möglich. Bevorzugt beträgt die Abweichung 25 % oder weniger, besonders bevorzugt 15 % oder weniger.

Die Mikropartikel können einen mittleren Durchmesser Dn (Zahlenmittelwert) von 1 nm bis 100 µm, vorzugsweise von 50 nm bis 10 µm, und besonders bevorzugt von 100 nm bis 3 µm aufweisen.

Die Oberfläche der sphärischen Partikel kann makroskopisch mit einer Himbeere verglichen werden, wobei die Tiefe von Unregelmäßigkeiten auf der Partikeloberfläche, wie Eindellungen oder Einschnitte, maximal 20 %, vorzugsweise 10 %, des mittleren Durchmessers der sphärischen Mikropartikel beträgt.

Die spezifische Oberfläche der Mikropartikel beträgt im allgemeinen von 1 m²/g bis 100 m²/g, vorzugsweise 1,5 m²/g bis 20 m²/g und besonders bevorzugt 3 m²/g bis 10 m²/g.

Weiter zeigen die erfindungsgmäßen Partikel vorzugsweise eine Dispersität D = Gewichtsmittetwert des Durchmessers (d_{w}) / Zahlenmittelwert des Durchmessers (dₙ) von 1,0 bis 10,0, insbesondere von 1,5 bis 5,0 und besonders bevorzugt von 2,0 bis 3,0.

Die hier benutzten Mittelwerte sind wie folgt definiert:
dₙ = Summe nᵢ x dᵢ / Summe ni = Zahlenmittelwert
d_{w} = Summe nᵢ x dᵢ² / Summe nᵢ x dᵢ = Gewichtsmittelwert
   mit
nᵢ = Anzahl der Partikel mit Durchmesser dᵢ,
dᵢ = ein bestimmter Durchmesser,
i = fortlaufender Parameter.

Für die vorliegende Erfindung können auch Mikropartikel eingesetzt werden, deren Oberfläche modifiziert worden ist, z.B. durch Derivatisierung von funktionellen Gruppen, wie Hydroxylgruppen der Polyglucane.

Lineare wasserunlösliche Polyglucane im Sinne der vorliegenden Erfindung sind Polysaccharide, die aus Glucanen als monomeren Bausteinen derart aufgebaut sind, daß die einzelnen Bausteine stets in der gleichen Art miteinander verknüpft sind. Jede so definierte Grundeinheit oder Baustein hat genau zwei Verknüpfungen, jeweils eine zu einem anderen Monomer. Davon ausgenommen sind lediglich die beiden Grundeinheiten, die den Anfang bzw. das Ende des Polysaccharids bilden.

Diese haben nur eine Verknüpfung zu einem weiteren Monomer und bilden die Endgruppendes linearen Polyglucans.

Besitzt die Gundeinheit drei oder mehr Verknüpfungen, wird von Verzweigung gesprochen. Dabei ergibt sich aus der Anzahl der Hydroxylgruppen pro 100 Grundeinheiten, die nicht am Aufbau des linearen Polymerrückgrats beteiligt sind und die Verzweigungen ausbilden, der sogenannte Verzweigungsgrad.

Erfindungsgemäß weisen die linearen wasserunlöslichen Polyglucane einen Verzweigungsgrad von maximal 8 % auf, d.h. sie haben maximal 8 Verzweigungen auf 100 Grundeinheiten. Vorzugsweise ist der Verzweigungsgrad kleiner 4 % und insbesondere maximal 2,5 %.

Erfindungsgemäß sind Polyglucane deren Verzweigungsgrad in 6-position maximal 0,5 %, ist, Besonders beverzugt sind Polyglucane deren Verzweigungsgrad in den anderen Positionen, z. B. in 2- bzw. 3-Position, vorzugsweise jeweils maximal 2 % und insbesondere 1 % ist.

Für die Erfindung sind insbesondere Polyglucane geeignet, die keine Verzweigungen aufweisen, bzw. deren Verzweigungsgrad so minimal ist, daß er mit herkömmlichen Methoden nicht mehr nachweisbar ist.

Beispiele für bevorzugte wasserunlösliche lineare Polyglucane sind lineare Poly-D-glucane, wobei die Art der Verknüpfung unwesentlich ist, solange Linearität im Sinne der Erfindung vorliegt. Beispiele sind Poly-alpha-D-glucane, insbesondere Poly-1,4-alpha-D-glucan und Poly-1,3-beta-D-glucan, wobei Poly-1,4-α-D-glucan besonders bevorzugt ist.

Für die vorliegende Erfindung beziehen sich die Präfixe "alpha", "beta" oder "D" allein auf die Verknüpfungen, die das Polymerrückgrat ausbilden und nicht auf die Verzweigungen.
Unter dem Begriff "wasserunlösliches Polyglucan" werden für die vorliegende Erfindung Verbindungen verstanden, die nach der Definition des Deutschen

Arzneimittelbuches (DAB = Deutsches Arzneimittelbuch, Wissenschaftliche Verlagsgesellschaft mbH, Stuttgart, Govi-Verlag, Frankfurt, Auflage, 1987) entsprechend den Klassen 4 bis 7 unter die Kategorien "wenig lösliche", "schwer lösliche", sehr schwer lösliche" bzw. "praktisch unlösliche" Verbindungen fallen.

Im Fall der erfindungsgemäß verwendeten Polyglucane bedeutet dies, daß mindestens 98 % der eingesetzten Menge, insbesondere mindestens 99,5 %, unter Normalbedingungen (T = 25 °C +/- 20 %, p= 101325 Pascal +/- 20 %) in Wasser unlöslich ist (entsprechend den Klassen 4 bzw. 5).

Für die vorliegende Erfindung sind schwer lösliche bis praktisch unlösliche Verbindungen, insbesondere sehr schwer lösliche bis praktisch unlösliche Verbindungen, bevorzugt.

"Sehr schwer löslich" entsprechend Klasse 6 kann durch folgende Versuchsbeschreibung veranschaulicht werden:
Ein Gramm des zu untersuchenden Polyglucans werden in 1 I entionisierten Wasser auf 130° C unter einem Druck von 1 bar erhitzt. Die entstehende Lösung bleibt nur kurzzeitig über wenige Minuten stabil. Beim Erkalten unter Normalbedingungen fällt die Substanz wieder aus. Nach Abkühlung auf Raumtemperatur und Abtrennung mittels Zentrifugation können unter Berücksichtigung der experimentellen Verluste mindestens 66 % der eingesetzten Menge zurückgewonnen werden.

Die erfindungsgemäß eingesetzten Polyglucane können beliebigen Ursprungs sein, solange die vorstehend angegebenen Bedingungen in bezug auf die Begriffe "linear" und "wasserunlöslich" erfüllt sind.

Sie können natürlich oder auf biotechnischen Wege gewonnen sein.

Beispielsweise können sie aus natürlichen pflanzlichen oder tierischen Quellen durch Isolierung und/oder Aufreinigung erhalten werden.

Es können auch Quellen zum Einsatz kommen, die gentechnisch derart manipuliert worden sind, daß sie im Vergleich zu der unmanipulierten Quelle einen höheren Anteil an nicht oder vergleichsweise geringfügig verzweigten Polyglucanen enthalten.

Sie können durch enzymatische oder chemische Entzweigung aus nicht-linearen Poly-alpha-glucanen hergestellt worden sein.
Dabei können nicht-lineare Polyglucane, die Verzweigungen enthalten, derart mit einem Enzym behandelt werden, daß es zur Spaltung der Verzweigungen kommt, so daß nach ihrer Abtrennung lineare Poly-glucane vorliegen. Bei diesen Enzymen kann es sich beispielsweise um Amylasen, iso-Amylasen, Gluconohydrolasen, Cyclomaltodextrin-glucanotransferasen oder Pullulanasen handeln.

Biotechnische Methoden umfassen biokatalytische, auch biotransformatorische, oder fermentative Prozesse.

Lineare Polyglucane hergestellt durch Biokatalyse (auch- Biotransformation) im Rahmen dieser Erfindung bedeutet, daß das lineare Polyglucan durch katalytische Reaktion von monomeren Grundbausteinen wie oligomeren Sacchariden, z.B. von Mono- und/oder Disacchariden, hergestellt wird, indem ein sogenannter Biokatalysator, üblicherweise ein Enzym, unter geeigneten Bedingungen verwendet wird. Man spricht in diesem Zusammenhang auch von "in vitro Biokatalyse".

Linerare Poly-alpha-glucane aus Fermentationen sind im Sprachgebrauch der Erfindung lineare Poly-alpha-glucane, die durch fermentative Prozesse unter der Verwendung in der Natur vorkommende Organismen, wie Pilzen, Algen, Bazillen, Bakterien oder Protisten oder unter der Verwendung von in der Natur nicht vorkommender Organismen, aber unter Zuhilfenahme von gentechnischen Methoden allgemeiner Definition modifizierten natürlichen Organismen, wie Pilzen, Algen, Bazillen, Bakterien oder Protisten gewonnen werden oder unter Einschaltung und Mithilfe von fermentativen Prozessen gewonnen werden können. Man spricht in diesem Zusammenhang auch von "in vivo Biokatalyse".

Beispiele für derartige Mikroorganismen sind Piichia pastoris, Trichoderma Reseii, Straphylokkus Camosus, Escerichia Coli oder Aspergillus Niger.

Vorteilhafte Verfahren für die biotechnische Gewinnung sind z. B. in der WO 95/31553 oder der nicht vorveröffentlichten deutschen Patentanmeldung der Anmelderin mit amtlichen Aktenzeichen 198 27 978.5 beschrieben.

Gemäß der WO 95/31553 werden Amylosucrasen zur Herstellung von linearen wasserunlöslichen Polyglucanen wie Poly-1,4-α-D-glucan mittels eines biokatalytischen Verfahrens verwendet. Weitere geeignete Enzyme sind Polysaccharidsynthasen, Stärkesynthasen, Glycoltransferasen, 1,4-α-D-Glucantransferasen, Glycogensynthasen oder auch Phosphorylasen.

Es können auch modifizierte wasserunlösliche lineare Polyglucane eingesetzt werden, wobei die Polyglucane beispielsweise durch Veresterung und/oder Veretherung in einer oder mehreren nicht an der linearen Verknüpfung beteiligten Positionen chemisch modifiziert worden sein können. Im Fall der bevorzugten 1,4 verknüpften Polyglucane kann die Modifizierung in 2-, 3- und/oder 6-Position erfolgen.

Modifikation im Sinne der Erfindung bedeutet, daß die vorhandenen Hydroylgruppen, die nicht an der Verknüpfung beteiligt sind, chemisch verändert werden. Dies schließt eine Ringöffnung der Glucaneinheiten aus wie sie z.B. bei der oxidativen Carboxylierung oder der Hydrolyse erfolgt. Maßnahmen für derartige Modifizierungen sind dem Fachmann hinlänglich bekannt.

So können lineare Polyglucane wie z.B. Pullulane, die an sich wasserlöslich sind, durch Modifizierung wasserunlöslich gemacht werden.

Für die vorliegende Erfindung werden bevorzugt wasserunlösliche lineare Polyglucane eingesetzt, die in einem biotechnischen, insbesondere in einem biokatalytischen oder einem fermentativen, Prozeß hergestellt worden sind.

Im Gegensatz zu Polyglucanen, die aus natürlichen Quellen, wie Pflanzen, isoliert werden, weisen die hierbei erhaltenen linearen wasserunlöslichen Poly-glucane ein besonders homogenes Eigenschaftsprofil auf, z. B. in bezug auf die Molekulargewichtsverteilung, sie enthalten keine oder allenfalls nur in sehr geringen Mengen unerwünschte Nebenprodukte, die aufwendig abgetrennt werden müssen oder allergene Reaktionen auslösen könnten, und lassen sich exakt spezifiziert auf einfache Weise reproduzieren.
Insbesondere können mit biotechnischen Methoden wasserunlösliche lineare wasserunlösliche Polyglucane erhalten werden, wie z. B. die bevorzugten Poly-1,4-α-D-glucane, die keine Verzweigungen enthalten, bzw. deren Verzweigungsgrad unterhalb der Nachweisgrenze herkömmlicher analytischer Methoden liegt.

Weiter können die Polyglucane in Form sogenannter alpha-amylaseresistenter Polyglucane eingesetzt werden wie sie am Beispiel von Poly-1,4-a-D-glucan in der, nicht vorveröffentlichten deutschen Patentanmeldung mit amtlichen Aktenzeichen 198 30 618.0 der Anmelderin beschrieben sind.

Alpha-amylaseresistente Polyglucane können durch Herstellung einer Suspension oder Dispersion aus wasserunlöslichen Polyglucanen und Wasser, Erwärmen der Suspension oder Dispersion auf eine Temperatur im Bereich von 50 bis 100°C, Abkühlenlassen der erhaltenen kleisterartigen Mischung auf eine Temperatur im Bereich von 50 °C bis an den Gefrierpunkt, vorzugsweise 35 bis 15 °C, 27 bis 22 °C, 16 bis 0 °C oder 6 bis 2 °C, über einen Zeitraum von 1 bis 72 h, vorzugsweise 1 bis 36 h und insbesondere 15 bis 30 h und Retrogradation der kleisterartigen Mischung bei einer gegenüber der Temperatur der erwärmten kleisterartigen Mischung erniedrigten Temperatur in einem Temperaturbereich von 90 bis 4 °C sowie gegebenenfalls Trocknung oder Entwässerung des erhaltenen Produktes erhaltenen werden.

Das Polyglucan kann auch als thermoplastisches Polyglucan eingesetzt werden, das erhältlich ist durch Aufschmelzen von linearem wasserunlöslichen Polyglucan und Hinzufügen von mindestens 20 Gew.%, vorzugsweise mindestens 30 Gew.%, eines Weichmachers wie Sorbitol, Glycerin, deren Kondensationsprodukte und Oligomere, DMSO, Bemsteinsäure, Citronensäure-Monohydrat, Apfelsäure, Weinsäure etc. bei ca. 170 °C.

Eine Beschreibung von geeigneten Maßnahmen und Eigenschaften von thermoplastischen Polyglucanen am Beispiel des bevorzugten linearen wasserunlöslichen Poly-1,4-α-D-glucans gibt die prioritätsältere nicht vorveröffentlichte deutsche Patentanmeldung mit amtlichen Aktenzeichen 198 52 826, auf die hierfür ausdrücklich bezug genommen wird.

Die Molekulargewichte M_{w} (Gewichtsmittel, bestimmt mittels Gelpermeationschromatographie im Vergleich zu einer Eichung mit Pullulanstandard) der erfindungsgemäß verwendeten linearen Polyglucane können in einem weiten Bereich von 0,75 x 10² g/mol bis 10⁷ g/mol variieren. Bevorzugt liegt das Molekulargewicht M_{w} in einem Bereich von 10³ g/mol bis 10⁶ g/mol und besonders bevorzugt von 10³ g/mol bis 10⁵ g/mol. Ein weiterer vorteilhafter Bereich ist von 2 x 10³ bis 8 x 10³. Entsprechende Bereiche gelten für das bevorzugt eingesetzte Poly(1,4-α-D-glucan).

Die Molekulargewichtsverteilung bzw. Polydispersität M_{w}/Mₙ kann ebenfalls in weiten Bereichen je nach Herstellungsverfahren des Polyglucans variieren.
Bevorzugte Werte sind von 1,01 bis 50, insbesondere von 1,01 bis 15. Besonders bevorzugt sind Polyglucane mit kleiner Polydispersität, z.B. von 1,01 bis 2.5.

Dabei nimmt die Polydispersität mit einer bimodalen Verteilung der Molekulargewichte zu.

Für die Herstellung der Mikropartikel kann ein einziges Polyglucan, insbesondere Poly-1,4-D-glucan und ganz besonders Poly-1,4-α-D-glucan oder Mischungen aus zwei oder mehreren Vertretern verwendet werden.

In einer weiteren Ausführungsform kann ein wasserunlösliches verzweigtes Polysaccharid, vorzugsweise ein Polyglucan, insbesondere ein Poly-1,4-alpha-D-glucan oder ein Poly-1,3-beta-D-glucan, zugesetzt werden.
Es können auch Gemische aus zwei oder mehreren verzweigten Polysacchariden zugegeben werden.

Die verzweigten Polysaccharide können beliebigen Ursprungs sein. In diesem Zusammenhang wird auf die diesbezüglichen Erläuterungen für die linearen wasserunlöslichen Polyglucane verwiesen. Bevorzugte Quellen sind Stärke und Stärkeanaloga wie Glykogen. Falls erforderlich kann in den verzweigten Polysacchariden der Anteil an linearen Strukturen durch geeignete Anreicherungsverfahren erhöht werden.

Für die Wasserunlöslichkeit gelten die gleichen Angaben wie für das lineare wasserunlösliche Polyglucan, das Molekulargewicht kann für die verzweigten Polysaccharide auch höher liegen, z. B. Werte bis vorzugsweise 10⁹ g/mol und mehr aufweisen.

Es können auch andere Polymere, insbesondere biokompatible oder bioabbaubare Polymere, beigemischt werden. Dabei hängt die Menge des oder der anderen Polymeren, die beigemengt werden, ohne daß die sphärische Gestalt und/oder sonstige Eigenschaften der herzustellenden Mikropartikel verändert werden, stets von dem zugesetzten Polymer ab.

Zur Sicherstellung der gewünschten Eigenschaften der Mikropartikel sollte der Anteil an linearen wasserunlöslichen Polyglucan mindestens 70 Gew.%, insbesondere 80 Gew.% und vorzugsweise 90 Gew.%, bezogen auf den Gesamtgehalt an linearem wasserunlöslichen Polyglucan inkl. ggf. verzweigtes Polysaccharid und gegebenenfalls weitere Polymere, betragen.

Gemäß einer besonders bevorzugten Ausführungsform bestehen die Mikropartikel zu 100 Gew.% aus linearem wasserunlöslichen Polyglucan, insbesondere linearen wasserunlöslichen Poly-1,4-α-D-glucan, das vorzugsweise biokatalytisch erhalten worden ist.

Beispiele für Verfahren zur Herstellung der Mikropartikel sind der Fällprozeß oder Sprühtrocknungsverfahren.

Die Herstellung der sphärischen Mikropartikel kann durch Lösen des wasserunlöstlichen linearen Polyglucans oder einer Mischung von mehreren davon sowie gegebenenfalls weiterer Polymere in einem Lösungsmittel , z. B. DMSO, Einbringen der Lösung in ein Fällmittel, z. B. Wasser, vorzugsweise bei einer Temperatur von 20° C bis 60° C, bei Bedarf Kühlen der Lösung auf eine Temperatur von minus 10° C bis plus 10° C und Abtrennen der dabei gebildeten Teilchen erfolgen.

Hierbei kann der Lösevorgang des als Ausgangsmaterial verwendetem Polyglucans bei Raumtemperatur oder höheren Temperaturen erfolgen.

Die Konzentration an linearem wasserunlöslichem Polyglucan inkl. ggf. verzweigtem Polysaccharrid und weiteren Polymeren in dem Lösungsmittel kann je nach Bedarf in weiten Grener variieren. Vorzugsweise liegt sie in einem Bereich von 0,02 g /ml bis 1,0 g/ml, insbesondere von 0,05 g/ml bis 0,8 g/ml und besonders bevorzugt von 0,3 g/l bis 0,6 g/l.

Beispiele für Fällmittel sind Wasser, Dichlormethan, ein Gemisch aus Wasser und Dichlormethan, Gemische aus Wasser und Alkoholen wie Methanol, Ethanol, Isopropanol, wobei Wasser sowie ein Gemisch aus Wasser und Dichlomethan besonders bevorzugt sind.

Vorzugsweise wird das Verhältnis Lösungsmittel zu Fällmittel in einem Bereich von 1 : 1000 bis 1:4 (Teil Lösungsmittel / Teile Fällmittel), vorzugsweise 1 : 100 bis 1 : 10 und insbesondere 1 : 70 bis 1 : 30 ausgewählt.

Im allgemeinen ist es dabei unerheblich, in welcher Reihenfolge das Lösungsmittel und das Fällmittel zusammengebracht werden, z. B. ob das Fällmittel zum Lösungsmittel oder umgekehrt gegeben wird.
Wichtig ist jedoch, daß eine schnelle Durchmischung gewährleistet wird.

Der Fällprozeß kann relativ langsam bei tiefer Temperatur über Nacht durchgeführt werden.

Er kann durch Variation der Temperatur und des Fällmittels beeinflußt und gesteuert werden.
Falls gekühlt wird, muß sichergestellt sein, daß das Gemisch aus Lösungsmittel und Fällmittel liquide bleibt und nicht erstarrt.

Durch Mitverwendung geeigneter Zusatzstoffe läßt sich auf die Eigenschaften der Mikropartikel wie Größe, Oberflächenstruktur, Porosität etc. sowie auf die Prozeßführung Einfluß nehmen.

Geeignete Zusatzstoffe sind z. B. oberflächenaktive Stoffe wie Natriumdodecylsulfat, N-Methylgluconamid, Polysorbate (z. B. Tween (eingetragene Marke)). Alkytpolyglycolether, Ethylenoxid-Propylenoxid-Blockpolymere (z. B. Pluronic (eingetragene Marke)), Alkylpolyglycolethersulfate, generell Alkylsulfate und Fettsäureglycolester, und Zucker wie z. B. Fructose, Saccharose, Glucose, wasserlösliche Cellulose oder heißwasserlösüches Poly-alpha-D-Glucan wie z. B. native oder chemisch modifizierte Stärken, aus diesen Stärken gewonnene Poly-alpha-DGlucane sowie stärkeanaloge Verbindungen.

Üblicherweise werden diese Zusatzstoffe dem Fällmittel zugesetzt. Die verwendete Menge hängt von dem jeweiligen Einzelfall sowie den erwünschten Partikeleigenschaften ab, wobei die Bestimmung der jeweils vorteilhaften Menge dem Fachmann geläufig ist.

Durch Zusatz von wasserlöslichen Cellulosederivaten zu dem Fällmittel lassen sich Mikropartikel mit besonders glatter Oberfläche erhalten, wobei die Tiefe der Unregelmäßigkeiten auf der Oberfläche der Mikropartikel im allgemeinen maximal 10 % des mittleren Durchmessers beträgt.

Beispiele von wasserlöslichen Cellulosederivaten sind Celluloseester und Celluloseether, deren Mischformen wie z.B. Hydroxypropylmethylcellulosen, Hydroxyethylcellulosen, Carboxymethylcellulosen, Celluloseacetate, Cellulosebutyrate, Cellulosepropionate, Cellutoseacetobutyrate, Celluloseacetopropionate, Cellulosenitrate, Ethylcellulosen, Benzylcellulosen, Methylcellulosen etc..

Es können auch Mischungen von verschiedenen wasserlöslichen Cellulosederivaten eingesetzt werden.

Unter dem Begriff "wasserlösliche Cellulosederivate" werden für die vorliegende Erfindung Verbindungen verstanden, die nach der Definition des Deutschen Arzneimittelbuches (DAB = Deutsches Arzneimittelbuch, Wissenschaftliche Verlagsgesellschaft mbH, Stuttgart, Govi-Verlag GmbH, Frankfurt, 9. Auflage, 1987) unter die Kategorie sehr leicht löslich bis schwer löslich fallen.

Die Konzentration des wasserlöslichen Cellulosederivats in dem Fällmittel ist nicht weiter kritisch. Die Obergrenze ergibt sich zwangsläufig aus der resultierenden Vikosität und damit der Verarbeitbarkeit der entstehenden Lösung.

Als vorteilhaft haben sich Konzentrationen von 2 g (Cellulosederivat)/l (Fällmittel) bis 150 g/l, vorzugsweise von 5 g/l bis 80 g/l und insbesondere 8 g/l bis 20 g/l, erwiesen.

Der Anteil an besonders kleinen Partikeln mit einem mittleren Durchmesser von 1 nm bis 2 µm kann gesteigert werden, indem dem Fällmittel heißwasserlösliches Poly-alpha-D-glucan zugesetzt wird.

Es können hierfür dieselben Poly-alpha-D-glucanverbindungen eingesetzt werden wie sie auch im Zusammenhang mit dem linearen wasserunlöslichen Polyglucan genannt worden sind, soweit diese das Merkmal heißwasserlöslich erfüllen.

Bevorzugte Beispiele sind native oder chemisch modifizierte Stärken, aus diesen Stärken gewonnene Poly-alpha-D-glucane sowie stärkeanaloge Verbindungen.

Unter stärkeanalogen Verbindungen werden Verbindungen verstanden, die aus Poly-alpha-D-glucanen bestehen, aber nicht-pflanzlichen Ursprungs sind. Ein Beispiel hierfür ist Glykogen oder Dextran.
Die heißwasserlöslichen Poly-alpha-D-glucane können als Mischung aus einem linearen und einem verzweigten Anteil eingesetzt werden, wie sie z. B. in Stärke vorliegt. In diesem Fall sollte der Anteil an linearem Poly-alpha-D-glucan mehr als 15 Gew.%, vorzugsweise 50 bis 99,5 Gew.%, insbesondere 60 bis 90 Gew.% und ganz besonders bevorzugt 65 bis 80 Gew.%, bezogen auf die Gesamtmenge Polyalpha-D-glucan im Fällmittel, betragen.

Sie können aber auch aus verzweigten Strukturen bestehen, wie sie z. B. im Amylopektin oder im Glykogen vorliegen.

Im Rahmen der vorliegenden Erfindung bedeutet "heißwassertöslich", daß die Polyalpha-D-glucane bei Raumtemperatur im wesentlich unlöslich sind, wobei vorzugsweise der gleiche Maßstab wie für den Begriff "wasserunlöslich" in Zusammenhang mit linearen Polysacchariden gilt.
Unter dem Begriff "Lösung" bzw. "Löslichkeit" werden insbesondere auch Suspensionen bzw. die Ausbildung von Suspensionen verstanden wie sie bei der Lösung von Stärke auftreten.

Beispielsweise zeigen die erfindungsgemäß bevorzugten heißwasseriösüchen Stärken bei Raumtemperatur so gut wie keine Löslichkeit in Wasser, während die sogenannten kaltwasserlöslichen Stärken unter diesen Bedingungen leichter löslich sind.

Die heißwasserlöslichen Stärken sind insbesondere dadurch charakterisert, daß sie bei Erhitzen unter Eigendruck, z.B. in einem Autoklaven, auf eine Temperatur im Bereich von etwa 100 bis etwa 160 °C Lösungen bilden, wobei die jeweilige Temperatur von der Art der Stärke abhängt.

Beispielsweise kann Kartoffelstärke bei ca. 100°C bis zur völligen Auflösung gekocht werden, während Maisstärke eine Temperatur von ca. 125°C erfordert.

Für das erfindungsgemäße Verfahren werden die heißwasserlöslichen Poly-alpha-D-glucane dem Fällmittel vorzugsweise in maximaler Konzentration zugesetzt, d.h. es wird eine gesättigte Lösung hergestellt.
Weitere geeignete Bereiche sind von mehr als 0,001 Gew.% bis 10 Gew.%, bevorzugt von 0,01 bis 2 Gew.% und insbesondere von 0,05 Gew.% bis 0,5 Gew.%, bezogen auf die eingesetzte Menge an Fällmittel.

Im Fall von thermoplastischen Polyglucanen können die Zusatzstoffe vorteilhafterweise als Weichmacher oder in Ergänzung der Weichmacher in die thermoplastische Mischung eingemischt werden, so daß eine trockene Pulvermischung vorliegt, die dann zu den Mikropartikeln verarbeitet werden kann, wobei der Bildungsprozeß der Mikropartikel auch erst in der endgültigen Rezeptur unter Einmischung der thermoplastischen Polyglucane erfolgen kann.

Eine ausführliche Beschreibung der hier verwendeten Mikropartikel, ihrer Herstellung und der dafür einsetzbaren wasserunlöslichen linearen Polyglucane findet sich in den prioritätsälteren, nicht vorveröffentlichten deutschen Patentanmeldungen der Anmelderin mit Aktenzeichen 197 37 481.6, 198 03 415.6, 198 16 070.4, 198 30 618.0, 198 27 978.7 und 198 39 214.1, 198 39 216.8 und 198 39 212.5, auf die für die vorliegende Beschreibung bezug genommen wird. Die drei letzten genannten Anmeldungen befassen sich insbesondere mit der Modifizierung der Partikelbeschaffenheit wie Größe und Oberflächenrauhigkeit.

Je nach Verwendungszweck können die erfindungsgemäßen Zubereitungen geeignete weitere für den jeweiligen Zweck übliche inhaltsstoffe enthalten.

Beispiele für derartige inhaltsstoffe wie sie insbesondere auch für kosmetische Mittel verwendet werden können sind Emulgatoren, Öle, Wachse, Fette oder andere übliche Bestandteile einer kosmetischen Formulierung wie Alkohole, Polyole, Polymere, Schaumstabilisatoren, Elektrolyte, Öle, flüchtige Kohlenwasserstoffe, Siliconöle oder Siliconderivate, Wirkstoffe, Feuchthaltemittel, Füllstoffe, Farbpigmente, Glanzpigmente, Farbstoffe, UV-Filter, Parfümöle, Antioxidantien, Stabilisatoren, entzündungshemmende Zusätze, durchblutungsfördemde Zusätze, Konservierungsmittel, Bakterizide, desodorierend wirkende Substanzen; Antitranspirantien, Insektenrepellentien, Vitamine, Proteine, Mittel zum Verhindern von Schäumen, Verdickungsmittel, weichmachende Substanzen, anfeuchtende und/oder feuchthaltende Substanzen, usw.
Ein Verzeichnis von möglichen zugelassenen Inhaltsstoffen für kosmetische Mittel, wie sie prinzipiell auch für die vorliegende Erfindung Verwendung finden können, enthält die Broschüre "Kosmetika - Inhaltsstoffe - Funktionen", die vom Industrieverband Körperpflege- und Waschmittel e.V. und dem Fachverband der chemischen Industrie Österreichs, Berufsgruppe Körperpflegemittel, Frankfurt am Main / Wien, Juni 1998, herausgegeben worden ist.

Medizinische topische Zubereitungen enthalten in der Regel ein oder mehrere Medikamente in wirksamer Konzentration.

Zur Unterscheidung zwischen kosmetischer und medizinischer Anwendung und den entsprechenden Produkten werden auf die geltenden Bestimmungen von Deutschland verwiesen, wie sie z.B. in der Kosmetikverordnung oder dem Lebensmittel- und Arzneimittelgesetz niedergelegt sind.

Es versteht sich die medizinischen Zubereitungen dieselben lnhaltsstoffe wie sie vorstehend beispielhaft für die kosmetische Anwendung genannt worden sind enthalten können, soweit sie für medizinische Zwecke zugelassen sind.

Im allgemeinen werden die jeweiligen inhaltsstoffe den erfindungsgemäßen Zubereitungen in für den jeweiligen Verwendungszweck üblichen Anteilen zugesetzt.

Die erfindungsgemäß eingesetzten Mikropartikel eignen sich auch besonders gut als Trägermaterial für den Zubereitungen zuzusetzende Inhaltsstoffen.
Beispielsweise können kosmetische und/oder medizinische Wirkstoffe auf den Mikropartikel adsorbiert oder in diese eingekapselt vorliegen.

Dabei können die Mikropartikel für die retardierte Freisetzung der in sie eingebrachten Stoffe ausgestaltet sein. Diesbezüglich wird auch auf die nicht vorveröffentlichte Anmeldung der Anmelderin mit amtlichen Aktenzeichen 198 16 070.4 verwiesen.

Die Herstellung der erfindungsgemäßen Zubereitungen kann nach den üblichen dem Fachmann geläufigen Regeln erfolgen.
Die erfindungsgemäßen Zubereitungen können in beliebiger, für den jeweiligen Anwendungszweck geeigneter Formulierung vorliegen, z.B. als Emulsionen wie O/W-Emulsionen, W/O-Emulsionen, multiple Emulsionen z. B W/O/W-, O/W/O-, O/W/O/W-, W/O/W/0- Emulsionen etc., auf Wachsbasis, wasserfrei, Hydrodispersionen, Gele, Öle, wasserfreie Salben bzw. Salbengrundlagen usw.

Der Zusatz der erfindungsgemäß verwendeten Mikropartikel zu topischen Zubereitungen führt zu einer Erhöhung des Weichheitsgefühls auf der Haut.

Die Ursache für dieses weiche, glatte sensorische Gefühl auf der Haut wird in der regelmäßigen sphärischen Gestalt der Mikropartikel vermutet, die einen rollenden Effekt, ähnlich einem Kugellager, bewirkt. Sie eignen sich daher insbesondere auch als Füllstoffe für spezielle kosmetische Effekte, wenn z.B. ein besonders weicher, glatter pudriger Effekt erzielt werden soll, zudem verleihen sie der Haut ein sanftes mattes Erscheinungsbild ähnlich dem "soft focus Effekt" beim Photographieren.

Im Gegensatz zu vielen Pigmenten wie z.B. nicht mikronisiertes Titandioxid, weißeln die erfindungsgemäß eingesetzten Mikropartikel nicht auf der Haut, d.h. sie wirken transparent und können daher vorteilhaft derartige Pigmente ersetzen.

So können sie als Ersatz für Stoffe wie Talk oder Kaolin genommen werden, die aufgrund ihrer unregelmäßigen oder plättchenförmigen Gestalt zu einem stumpfen Gefühl beim Auftragen führen, z.B. in pigmentierten Produkten, oder als Kompaktierungshilfsmittel in gepressten Pudern eingesetzt werden.

Weiter wurde für die Mikropartikel ein absorbierender Effekt beobachtet.
Aufgrund dieses absorbierenden Effektes eignen sie sich besonders gut auch als Zusatzstoff in Deodorantien, Körperpuder wie Body Talc, zur Aufnahme von überschüssigen Hautfett z.B. in anti-oil- oder Antiakne-Produkten.

Weiter konnte beobachtet werden, daß sie Hautrauhigkeiten zu reduzieren vermögen, insgesamt einen beruhigenden Effekt auf die Haut haben, sowie eine weichmachende und moisturizierende Wirkung ausüben.

Neben den kosmetischen Effekten sind die erfindungsgemäß eingesetzten Mikropartikel hervorragend dispergierbar und bilden selbst ohne Zusatz von Dispergierhilfsmitteln stabile Dispersionen oder Suspensionen aus.

Eine gute Dispergierbarkeit ist von Vorteil, da auf den Zusatz von Dispergierhilfsrnitteln und ähnlichem verzichtet werden kann und besonders hautverträgliche Zubereitungen erhalten werden können, die zudem einfacher und daher billiger herzustellen sind.

Geeignete Anteile an Mikropartikeln für Cremes, Lotionen, Make-up, Kompaktcremes und älinlichem liegen im Bereich von etwa 0,5 Gew.0/o bis etwa 40 Gew.%, vorzugsweise etwa 2 bis etwa 10 Gew.%, für Puder von etwa 0,5 bis etwa 80 Gew.%, für Salben und pharmazeutische Salben können entsprechende Anteile eingesetzt werden (die Anteile sind jeweils auf das Gesamtgewicht der betreffenden Zubereitungttezogen).

Es verstehtsotr, daß bei Bedarf z. B. für spezielle Anwendungen auch mehr z.B. 100 Gew.% weniger Mikropartikel eingesetzt werden können.

Der Anteil anlikropartikeln in den Zubereitungen richtet sich selbstverständlich nach dem enunschten Effekt und / oder der speziellen Formulierung der Zubereitung.

Nachstehend wird die Erfindung anhand von einzelnen Beispielen veranschaulicht.

### Beispiele 1 und 2 und Vergleichsbeispiele 1 und 2

### Herstellvorschrift für Lotion und Creme

Die Zusammensetzung für die Lotion (Beispiele 1a, 1b und Vergleichsbeispiel 1) sowie die Creme (Beispiele 2a, 2b und Vergleichsbeispiel 2) sind in Tabelle 1 aufgeführt.

### Vorbereitung:

### Fettphase:

Emulgatoren und Öle unter Rühren auf 70° C erwärmen. Konservierungsmittel (Parabene) zugeben und unter Rühren lösen.

### Wasserphase:

Demin. Wasser auf 70° C erwärmen, restl. Bestandteile unter Rühren lösen. Mikropartikel dispergieren.

### Konservierungs-Lösung:

Konservierungsmittel (Imidazolidinyl Urea) in demin. Wasser lösen.

### Herstellung:

Fettphase vorlegen, t = 68-70° C. Wasserphase (t = 68-70° C) unter Rühren und Homogenisieren zugeben. Nach der Zugabe 20 min. rühren und homogenisieren, t = 65-68° C.

### Kühlen:

unter Rühren auf 40° C. Konservierungsmittel-Lösung und Parfümöl zugeben und 5 min. rühren und homogenisieren.
Unter Rühren auf 30°C kühlen.

Für die erfindungsgemäßen Cremes und Lotionen wurde gegenüber den Zubereitungen ohne Mikropartikel eine deutliche Verbesserung der Weichheit und Glätte sowie ein samtiges Gefühl beim Applizieren beobachtet.
Der Mikropartikelzusatz führte zu keinem Weißeln auf der Haut.

### Beispiele 3a und b sowie Vergleichsbeispiel 3

Herstellvorschrift für Compact Cream Make up Die Zusammensetzung ist in Tabelle 2 wiedergegeben.

### Vorbereitung:

Lanolin und Öle auf 80-82° C erwärmen. Antioxydant und filmbildner unter Rühren lösen. Vorgeschmolzene Wachse (t = 80° C) zugeben.

### Herstellung:

Vormischung aus Farbpigmenten, Titandioxid, Talkum, Mica und Mikropartikel unter Rühren und Homogenisieren zugeben.
Anschl. 30 min. rühren und homogenisieren, t = 82° C.

### Entleeren:

Bulk luftfrei in geeignete Behältnisse entleeren.

Im Vergleich zur Basisrezeptur zeigte die erfindungsgemäße Zubereitung eine reduzierte Wachsartigkeit. Der Zusatz von 5 Gew.% Mikropartikel bewirkte ein angenehmes pudriges Gefühl bei der Entnahme.

### Beispiele 4a und b und Vergleichsbeispiel 4

Herstellvorschrift für Foundation Die Zusammensetzung ist in Tabelle 3 wiedergegeben.

### Vorbereitung:

Emulgator, Konsistenzgeber und Öl auf 70° C erwärmen und mischen.
Konservierung zugeben und unter Rühren lösen.

Demin. Wasser und Propylenglycol auf 70° C erwärmen, Wirkstoff und Stabilisator unter Rühren lösen. Vormischung aus Farbpigmenten, Titandioxid und Mikropartikel bei laufendem Homogenisator einsaugen. Anschließend 30 min. rühren und homogenisieren.

### Herstellung:

Filtrierte Fettphase (t = 70° C) unter Rühren und Homogenisieren einsaugen und weitere 10 min. rühren und homogenisieren, t = 65° C.

### Kühlen:

unter Rühren auf 40° C. Vorlösung Konservierung in demin. Wasser und Parfümöl bei 40° C zugeben. Anschließend 10 min. rühren und homogenisieren.
Unter Rühren und Homogenisieren auf 30° C kühlen.

Im Vergleich zu der Grundierung ohne Mikropartikel fühlte sich die erfindungsgemäße Zubereitung deutlich cremiger an. Zudem wurde eine Viskositätssteigerung beobachtet.

**Tabelle 1**

| Komponente | Vergleiche | 1a | 1b | Vergleiche | 2a | 2b |
|---|---|---|---|---|---|---|
| Sorbitan Stearate | 1,50 | 1,50 | 1,50 | 1,50 | 1,50 | 1,50 |
| Polysorbate 60 | 2,00 | 2,00 | 2,00 | 2,00 | 2,00 | 2,00 |
| Cetyl Alcohol | | | | 2,50 | 2,50 | 2,50 |
| Mineral Oil | 8,00 | 8,00 | 8,00 | 8,00 | 8,00 | 8,00 |
| Decyl Oleate | 4,00 | 4,00 | 4,00 | 4,00 | 4,00 | 4,00 |
| Glyceryl Stearate | 4,00 | 4,00 | 4,00 | 5,00 | 5,00 | 5,00 |
| Dimethicone | 0,50 | 0,50 | 0,50 | 0,20 | 0,20 | 0,20 |
| Parabene | 0,35 | 0,35 | 0,35 | 0,35 | 0,35 | 0,35 |
| | | | | | | |
| | | | | | | |
| Vollentsaltzes Wasser (Aqua) | 63,30 | 63,30 | 63,30 | 58,60 | 58,60 | 58,60 |
| Allantoin | 0,20 | 0,20 | 0,20 | 0,20 | 0,20 | 0,20 |
| Propylenglycol | 3,50 | 3,50 | 3,50 | 5,00 | 5,00 | 5,00 |
| | | | | | | |
| | | | | | | |
| Vollentsalztes Wasser (Aqua) | 10,00 | 8,00 | 5,00 | 10,00 | 8,00 | 5,00 |
| Mikropartikel * | | 2,00 | 5,00 | | 2,00 | 5,00 |
| Vollentsaltzes Wasser (Aqua) | 2,00 | 2,00 | 2,00 | 2,00 | 2,00 | 2,00 |
| Imidazolidinyl Urea | 0,50 | 0,50 | 0,50 | 0,50 | 0,50 | 0,50 |
| | | | | | | |
| | | | | | | |
| Parfüm (Fragrance) | 0,15 | 0,15 | 0,15 | 0,15 | 0,15 | 0,15 |
| | | | | | | |
| | 100,00 | 100,00 | 100,00 | 100,00 | 100,00 | 100,00 |

| | | | | | | |
|---|---|---|---|---|---|---|
| *keine INCl Bezeichnung | | | | | | |

**Tabelle 2**

| Komponente (INCI) | Vergleiche 3 | 3a | 3b |
|---|---|---|---|
| Lanolin | 10,10 | 10,10 | 10,10 |
| Mineral Oil | 7,85 | 7,85 | 7,85 |
| Castor Oil | 2,80 | 2,80 | 2,80 |
| Octyldodecanol | 22,00 | 20,00 | 17,00 |
| Isopropyl Palmitate | 5,00 | 5,00 | 5,00 |
| Antioxidans | 0,05 | 0,05 | 0,05 |
| PVP/Eicosane Copolymer | 0,15 | 0,15 | 0,15 |
| Eisenoxid | 0,68 | 0,68 | 0,68 |
| Titandioxid * | 2,75 | 2,75 | 2,75 |
| Talcum * | 16,07 | 16.07 | 16,07 |
| Mica * | 13,90 | 13,90 | 13,90 |
| Mikropartikel * | | 2,00 | 5,00 |
| Cera Microcritallina | 9,70 | 9,70 | 9,70 |
| Petrolatum | 7,65 | 7,65 | 7,65 |
| Camauba | 1,30 | 1,30 | 1,30 |
| | | | |
| | 100,00 | 100,00 | 100,00 |

| | | | |
|---|---|---|---|
| * keine INCI Bezeichnung | | | |

**Tabelle 3**

| Komponente INCI | Vergleich 4 | 4a | 4b |
|---|---|---|---|
| Glyceryl Stearate | 9,00 | 9,00 | 9,00 |
| Isopropyl Myrisate | 2,25 | 2,25 | 2,25 |
| Cetyl Alcohol | 1,50 | 1,50 | 1,50 |
| Parabene | 0,35 | 0,35 | 0,35 |
| | | | |
| | | | |
| Vollentsalztes Wasser (Aqua) | 62,27 | 62,27 | 62,27 |
| Allantoin | 0,25 | 0,25 | 0,25 |
| Xanthan Gum | 0,30 | 0,30 | 0,30 |
| | | | |
| | | | |
| Propylenglycol | 8,90 | 8,90 | 8,90 |
| Eisenoxid | 0,68 | 0,68 | 0,68 |
| Titiandioxid * | 2,75 | 2,75 | 2,75 |
| | | | |
| | | | |
| Vollentsalztes Wasser (Aqua) | 10,00 | 8,00 | 5,00 |
| Mikropartikel | | 2,00 | 5,00 |
| | | | |
| | | | |
| Parfüm (Fragrance) | 0,25 | 0,25 | 0,25 |
| Vollentsalztes Wasser (Aqua) | 1,00 | 1,00 | 1,00 |
| lmidazolidinyl Urea | 0,50 | 0,50 | 0,50 |
| | | | |
| | 100,00 | 100,00 | 100,00 |

| | | | |
|---|---|---|---|
| *keine INCl Bezeichnung | | | |

### Beispiel 5

### Herstellung vom Mikropartikeln aus Poly-1,4-α-D-glucan

500 mg Poly-1,4-2-D-gulcan werden in 2,5 ml Dimethylsulfoxid (DMSO, p.a. von Riedel-de-Haen) bei ca. 70° C gelöst. Die DMSO-Lösung wird in 100 ml bidestilliertern Wasser unter Rühren eingetropft und die Lösung über Nacht bei 5° C aufbewahrt. Die feine milchige Suspension wird für 15 Minuten bei 3500 Umdrehungen pro Minute zentrifugiert und der Überstand abdekantiert. Der Bodensatz wird mit bidestilliertem Wasser aufgeschlämmt und erneut zentrifugiert. Der Vorgang wird noch zwei Mal wiederholt. Die Suspension wird im Anschluß gefriergetrocknet. Es werden 311 mg weißer Poty-1,4-α-D-glucanpartikel erhalten. Dies entspricht einer Ausbeute von 62 % farbloser Mikropartikel.

## Patentansprüche

1. Topische Zubereitung, die als wesentlichen Bestandteil sphärische Mikropartikel enthält, die ganz oder teilweise aus mindestens einem wasserunlöslichen linearen Polyglucan bestehen, wobei linear einen Verzweigungsgrad von nicht mehr als 0,5% in 6-Position bedeutet.

2. Topische Zubereitung nach Anspruch 1, **dadurch gekennzeichnet, daß** die sphärische Mikropartikel einen mittleren Durchmesser von 1 nm bis 100 µm haben.

3. Topische Zubereitung nach Anspruch 1 oder 2, **dadurch gekennzeichnet, daß** die Tiefe von Unregelmäßigkeiten auf der Partikeloberfläche maximal 20 % des mittleren Durchmessers der sphärischen Mikropartikel beträgt.

4. Topische Zubereitung nach einem der vorhergehenden Ansprüche, **dadurch gekennzeichnet, daß** die Mikropartikel in der topischen Zubereitung in einer Menge von 0,5 bis 80 Gew. % enthalten sind, bezogen auf das Gesamtgewicht der topischen Zubereitung.

5. Topische Zubereitung nach einem der vorhergehenden Ansprüche, **dadurch gekennzeichnet, daß** das mindestens eine wasserunlösliche Polyglucan Poly-1,4-α-D-glucan und/oder Poly-1,3-β-D-glucan, insbesondere Poly-1,4-α-D-glucan, ist.

6. Topische Zubereitung nach einem der vorhergehenden Ansprüche, **dadurch gekennzeichnet, daß** das mindestens eine wasserunlösliche lineare Polyglucan nach einer biotechnischen Methode erhalten worden ist.

7. Topische Zubereitung nach einem der vorhergehenden Ansprüche, **dadurch gekennzeichnet, daß** das mindestens eine wasserunlösliche lineare Polyglucan biokatalytisch erzeugt worden ist

8. Topische Zubereitung nach einem der vorhergehenden Ansprüche, **dadurch gekennzeichnet, daß** der Anteil an wasserunlöslichen linearen Polyglucan in den Mikropartikeln mindestens 70 % beträgt, bezogen auf den Gesamtgehalt an Polyglucan inkl. ggf. verzweigtem Polysaccharid und weiteren Polymeren.

9. Topische Zubereitung nach einem der vorhergehenden Ansprüche, **dadurch gekennzeichnet, daß** die Mikropartikel zu 100 % aus mindestens einem wasserunlöslichen linearen Polyglucan bestehen.

10. Topische Zubereitung nach einem der vorhergehenden Ansprüche, **dadurch gekennzeichnet, daß** die topische Zubereitung ein kosmetisches Mittel wie Körperpflegemittel oder eine dekorative Kosmetik ist.

11. Topische Zubereitung nach einem der Ansprüche 1 bis 9, **dadurch gekennzeichnet, daß** die topische Zubereitung eine human- oder tiermedizinische Zubereitung zur äußeren Applikation ist.

12. Topische Zubereitung nach einem der vorhergehenden Ansprüche, **dadurch gekennzeichnet, daß** die topische Zubereitung ausgewählt ist unter Cremes, Kompaktcremes, Lotionen, Masken, Puder, Salben, Salbengrundlagen und Seifen.

13. Topische Zubereitung nach Anspruch 10, **dadurch gekennzeichnet, daß** die topische Zubereitung für die dekorative Kosmetik ist und ausgewählt ist unter Cremes, Puder oder Fonds für Make-up.

14. Topische Zubereitung nach Anspruch 13, **dadurch gekennzeichnet, daß** das Make-up Rouge, Lidschatten oder Lippenstift ist.

15. Verwendung von Mikropartikeln, die ganz oder teilweise aus mindestens einem wasserunlöslichen linearen Polyglucan bestehen, in topischen Zubereitungen, wobei der Verzweigungsgrad des Polyglucans in 6-Position 0,5% nicht überschreitet..

## Claims

1. A topical preparation which, as an essential constituent, contains spherical microparticles which consist entirely or partially of at least one water-insoluble linear polyglucan, wherein linear means a degree of branching of not more than 0,5 % in 6-position.

2. The topical preparation as claimed in claim 1, **characterized in that** the spherical microparticles have a mean diameter of 1 nm to 100 µm.

3. The topical preparation as claimed in claim 1 or 2, **characterized in that** the depth of irregularities an the particle surface is at most 20% of the mean diameter of the spherical microparticles.

4. The topical preparation as claimed in one of the preceding claims, **characterized in that** the microparticles are contained in the topical preparation in an amount from 0.5 to 80% by weight, based an the total weight of the topical preparation.

5. The topical preparation as claimed in one of the preceding claims, **characterized in that** the at least one water-insolubie polyglucan is poly-1,4-a-D-glucan and/or poly-1,3-β-D-glucan, in particular polyl,4-a-D-glucan.

6. The topical preparation as claimed in one of the preceding claims, **characterized in that** the at least one, water-insoluble linear polyglucan has been obtained by a biotechnological method.

7. The topical preparation as claimed in one of the preceding claims, **characterized in that** the at least one water-insoluble linear polyglucan has been produced biocatalytically.

8. The topical preparation as claimed in one of the preceding claims, **characterized in that** the proportion of water-insoluble linear polyglucan in the microparticles is at least 70%, based on the total content of polyglucan including optionally branched polysaccharide and further polymers.

9. The topical preparation as claimed in one of the preceding claims, **characterized in that** the microparticles consist to 100% of at least one water-insoluble linear polyglucan.

10. The topical preparation as claimed in one of the preceding claims, **characterized in that** the topical preparation is a cosmetic composition such as bodycare compositions or a decorative cosmetic.

11. The topical preparation as claimed in one of claims 1 to 9, **characterized in that** the topical preparation is a human or veterinary medicinal preparation for external application.

12. The topical preparation as claimed in one of the preceding claims, **characterized in that** the topical preparation is selected from creams, compact creams, lotions, masks, powders, ointments, ointment bases and soaps.

13. The topical preparation as claimed in claim 10, **characterized in that** the topical preparation is for decorative cosmetics and is selected from creams, powder or foundations for make-up.

14. The topical preparation as claimed in claim 13, **characterized in that** the make-up is rouge, eyeshadow or lipstick.

15. The use of microparticles which consist entirely or partially of at least one water-insoluble linear polyglucan in topical preparations, wherein the degree of branching of the polyglucan in not more than 0,5 % in 6-Position.

## Revendications

1. Préparation topique qui contient comme constituant essentiel des microparticules sphériques qui consistent en totalité ou en partie en au moins un polyglucane linéaire insoluble dans l'eau, où linéaire signifie un degré de ramification ne dépassant pas 0,5 % en position 6.

2. Préparation topique selon la revendication 1 **caractérisée en ce que** les microparticules sphériques ont un diamètre moyen de 1 nm à 100 µm.

3. Préparation topique selon la revendication 1 ou 2 **caractérisée en ce que** la profondeur des irrégularités sur la surface des particules représente au maximum 20 % du diamètre moyen des microparticules sphériques.

4. Préparation topique selon l'une des revendications précédentes **caractérisée en ce que** les microparticules dans la préparation topique sont contenues en une quantité de 0,5 à 80 % en poids par rapport au poids total de la préparation topique.

5. Préparation topique selon l'une des revendications précédentes **caractérisée en ce que** le ou les polyglucanes insolubles dans l'eau est ou sont le poly-1,4-α-D-glucane et/ou le poly-1,3-β-D-glucane, en particulier le poly-1,4-α-D-glucane.

6. Préparation topique selon l'une des revendications précédentes **caractérisée en ce que** le ou les polyglucanes linéaires insolubles dans l'eau ont été obtenus selon une méthode biotechnique.

7. Préparation topique selon l'une des revendications précédentes **caractérisée en ce que** le ou les polyglucanes linéaires insolubles dans l'eau ont été produits biocatalytiquement.

8. Préparation topique selon l'une des revendications précédentes **caractérisée en ce que** la proportion de polyglucane linéaire insoluble dans l'eau dans les microparticules est d'au moins 70 %, par rapport à la teneur totale en polyglucane y compris un polysaccharide éventuellement ramifié et d'autres polymères.

9. Préparation topique selon l'une des revendications précédentes **caractérisée en ce que** les microparticules consistent à 100 % en au moins un polyglucane linéaire insoluble dans l'eau.

10. Préparation topique selon l'une des revendications précédentes **caractérisée en ce que** la préparation topique est un agent cosmétique comme un agent de soins corporels ou un cosmétique décoratif.

11. Préparation topique selon l'une des revendications 1 à 9 **caractérisée en ce que** la préparation topique est une préparation de médecine humaine ou vétérinaire pour l'application externe.

12. Préparation topique selon l'une des revendications précédentes **caractérisée en ce que** la préparation topique est choisie parmi les crèmes, les crèmes compactes, les lotions, les masques, les poudres, les pommades, les bases de pommades et les savons.

13. Préparation topique selon la revendication 10 **caractérisée en ce que** la préparation topique est pour un cosmétique décoratif et est choisie parmi les crèmes, les poudres ou les fonds pour maquillage.

14. Préparation topique selon la revendication 13 **caractérisée en ce que** le maquillage est un rouge, une ombre à paupières ou un bâton de rouge à lèvres.

15. Utilisation de microparticules qui consistent en totalité ou en partie en au moins un polyglucane linéaire insoluble dans l'eau, dans des préparations topiques, où le degré de ramification du polyglucane en position 6 ne dépasse pas 0,5 %.
